# EUROPEAN PATENT APPLICATION

(11) **EP 4 497 470 A1**
(43) Date of publication of application: **29.01.2025**
(21) Application number: 22933571.6
(22) Date of filing: 21.07.2022
(51) Int. Cl.: A61N 5/10

(54) **CHARGED PARTICLE BEAM DEFLECTION DEVICE**

(30) Priority: 23.03.2022 JP 2022046420; 30.03.2022 JP 2022056566
(71) Applicant: B dot Medical Inc., Tokyo 134-0003 (JP)
(72) Inventor: TAKI Yoshiaki, Tokyo 134-0003 (JP); TAKADA Yuya, Tokyo 134-0003 (JP); ISHIHARA Shun, Tokyo 134-0003 (JP)
(74) Representative: Algemeen Octrooi- en Merkenbureau B.V.
(86) International application number: PCT/JP2022/028317
(87) International publication number: WO 2023/181434

(57) **Abstract**

A charged particle beam deflection device includes a layered structure including a plurality of layers layered in a direction from an interior toward an exterior and having a hollow shape through which a charged particle beam passes, a first coil configured to deflect the charged particle beam in a first direction different from a travel direction of the charged particle beam, a second coil configured to deflect the charged particle beam in a second direction different from the first direction, and a pressing portion located on an outer peripheral surface of the layered structure and configured to press the outer peripheral surface from the exterior toward the interior. The plurality of layers include a first coil support layer configured to support the first coil and a second coil support layer configured to support the second coil. The layered structure includes a gap, through which a cooling solvent can flow, between two adjacent layers among the plurality of layers.

## Description

### Technical Field

The present invention relates to a charged particle beam deflection device.

### Background Art

Particle beam therapy has been used to treat patients by irradiating an affected area, such as a cancer area, of a patient with a charged particle beam accelerated to high energy.

Patent Document 1 discloses a technique in which a charged particle beam generated by using a beam generation device is accelerated by a beam acceleration device, a trajectory of the charged particle beam is adjusted by a beam scanning device, and the affected area of the patient is irradiated with the charged particle beam. The beam scanning device described in Patent Document 1 includes a plurality of coils that deflect the charged particle beam and a structural member formed by layering a plurality of layers that support the plurality of coils. Patent Document 1 describes making a cooling solvent flow through gaps in the structural member to cool the coils.

### Citation List

### Patent Literature

Patent Document 1: JP 2021-32611 A

### Summary of Invention

### Technical Problem

(1) However, the present inventors have recognized the following problem. That is, although the structural member needs to be formed by layering the plurality of layers with high accuracy, in the technique described in Patent Document 1, a layer constituting the structural member may deviate in position when the cooling solvent is made to flow through the gaps in the structural member. This results in difficulties in securing the water channel as designed. In the technique described in Patent Document 1, layer deviation is not taken into consideration and thus, when the cooling solvent flows, a layer may deviate in position, leading to failure to make the cooling solvent flow sufficiently smoothly. As a result, more heat than expected is generated in the coils, making it difficult to achieve the desired therapeutic irradiation.
(2) The present inventors have also recognized the following problem. That is, the present inventors considered that, while the technique described in Patent Document 1 forms a flow channel through which the cooling solvent flows in the gaps of the structural member, there is room for improvement in cooling the coils more efficiently with the cooling solvent.

The present invention has been made in view of the above circumstances, and (1) one exemplary object of the present invention is to provide a charged particle beam deflection device that enables a cooling solvent for cooling a coil to flow smoothly.

(2) Another exemplary object is to provide a charged particle beam deflection device that enables more efficient cooling of a coil.

### Solution to Problem

(1) To solve the problems described above, a charged particle beam deflection device according to an aspect of the present invention includes a layered structure having a hollow shape where a charged particle beam passes through an interior of the layered structure and including a plurality of layers layered in a direction from the interior toward an exterior of the layered structure, a first coil configured to deflect the charged particle beam in a first direction different from a travel direction of the charged particle beam, a second coil configured to deflect the charged particle beam in a second direction different from the first direction, and a pressing portion located on an outer peripheral surface of the layered structure and configured to press the outer peripheral surface from the exterior toward the interior. The plurality of layers include a first coil support layer supporting the first coil and a second coil support layer supporting the second coil. The layered structure includes a gap, through which a cooling solvent flows, between two adjacent layers among the plurality of layers.
(2) A charged particle beam deflection device according to an aspect of the present invention includes a hollow member having a hollow shape through which a charged particle beam passes, a layered structure including a plurality of layers covering an outer peripheral surface of the hollow member and layered in a direction from an interior toward an exterior of the hollow member, a first coil configured to deflect the charged particle beam in a first direction different from a travel direction of the charged particle beam, and a second coil configured to deflect the charged particle beam in a second direction different from the first direction. The plurality of layers include a first coil support layer supporting the first coil and a second coil support layer supporting the second coil. The hollow member is disposed between an inner peripheral surface of the layered structure and the outer peripheral surface of the hollow member, forming a gap in which the outer peripheral surface of the hollow member does not come into contact with the first coil and the second coil.

Note that any combination of the constituent elements described above and a conversion of expression of the present invention between methods, devices, systems, and the like are also effective as aspects of the present invention.

### Advantageous Effects of Invention

(1) According to the present invention, it is possible to provide a charged particle beam deflection device capable of making a cooling solvent for cooling a coil flow smoothly.
(2) According to the present invention, it is possible to provide a charged particle beam deflection device capable of cooling a coil more efficiently.

### Brief Description of Drawings

FIG. 1 is a schematic configuration view of a charged particle beam irradiation device according to an embodiment of the present invention.
FIG. 2 is a perspective view of a charged particle beam deflection device according to the same embodiment.
FIG. 3 is a cross-sectional view of the charged particle beam deflection device according to the same embodiment.
FIG. 4 is an enlarged view of a region A illustrated in FIG. 3.
FIG. 5 is a view illustrating a case in which a hole having a form different from that in the example illustrated in FIG. 4 is formed in an inflow member.
FIG. 6 is an enlarged view of a region B illustrated in FIG. 3.
FIG. 7 is a view of a cross section of a structure, the cross section being orthogonal to an X-axis direction.
FIG. 8 is a perspective view of an inner tube according to the embodiment of the present invention.
FIG. 9 is a perspective view of a first winding frame layer disposed on the inner tube.
FIG. 10 is a perspective view illustrating a water channel member of a first water channel layer according to the embodiment of the present invention.
FIG. 11 is a perspective view of a second winding frame layer according to the same embodiment.
FIG. 12 is a perspective view of an outer tube according to the same embodiment.
FIG. 13 is an enlarged view illustrating part of a cross section of the inner tube and the structure, the cross section being orthogonal to an X-axis.
FIG. 14 is an enlarged view illustrating part of a cross section of the inner tube and the structure according to a first modification, the cross section being orthogonal to the X-axis.
FIG. 15 is an enlarged view illustrating part of a cross section of the inner tube and the structure according to a second modification, the cross section being orthogonal to the X-axis.
FIG. 16 is a schematic configuration view of a charged particle beam deflection device according to a second embodiment.
FIG. 17 is a view of the structure in a positioned state, as viewed from an emission side of the charged particle beam.
FIG. 18 is an enlarged view illustrating a state in which the structure is positioned by positioning pins.

### Description of Embodiments

Hereinafter, embodiments of the present invention will be described in detail with reference to the drawings. Note that, in the descriptions of the drawings, the same elements are denoted by the same reference numerals or signs, and redundant description thereof will be omitted as appropriate. Further, the configurations described below are exemplary and do not limit the scope of the present invention in any way.

### Background

A scanning electromagnet, which is an electromagnet for scanning a charged particle beam, has a role of ultimately controlling a position of a charged particle beam with which a human body is irradiated. An installation position of the scanning electromagnet therefore requires high precision. If this requirement is not satisfied, deviation occurs in the position irradiated with the charged particle beam, lowering the precision of the treatment. In actual practice, in the field of treatment using scanning electromagnets, when deviation occurs in the position irradiated with a charged particle beam, the deviation is corrected by a system related to irradiation control, thereby enhancing the precision of the treatment. While the deviation in the irradiation position is ultimately resolved, the workload associated with this correction is substantial. Installing the scanning electromagnet in the appropriate position to reduce this workload is a significant challenge.

Further, from a facility management perspective, the return on investment for a treatment device is a major concern for operators of treatment facilities. After the introduction of the treatment device, there is a demand to put the treatment device into operation as quickly as possible to advance the return on the investment. Merely installing the treatment device does not enable its use for treatment. The treatment device needs to be adjusted in terms of both hardware and software to obtain a charged particle beam with the precision required for treatment. In this process, accurately installing the physical components of the treatment device leads to a reduction in the time for carrying out this adjustment, which naturally also leads to an increase in the number of patients who can receive treatment.

In embodiments of the present invention described below, two pairs of scanning electromagnets are arranged in parallel and the diameters of the scanning electromagnets are inclined, reducing the size of the charged particle beam deflection device. Size reduction of the charged particle beam deflection device is directly linked to ease of handling. If the charged particle beam deflection device can be reduced in size, improvements in workability in adjusting the irradiation of the charged particle beam can be achieved. In particular, size reduction of the device significantly impacts workability when installing (introducing) the device.

Generally, making the device smaller in an aperture diameter direction results in fewer coil turns, which leads to a smaller magnetic field generated by the scanning electromagnet. Similarly. when the device is made smaller in an axial length direction as described above, the magnetic field generated by the scanning electromagnet also weakens. As the magnetic field weakens, the force that bends the charged particle beam diminishes. When this force diminishes, in order to maintain the width of the irradiation field, it becomes necessary to increase a distance between the scanning electromagnet and an isocenter to compensate for the weakened magnetic field. In a case in which the charged particle beam is emitted from a plurality of directions by moving the scanning electromagnet circumferentially around the isocenter and the distance between the scanning electromagnet and the isocenter becomes long, the size of the device increases. This is because the distance between the scanning electromagnet and the isocenter becomes the radius of the circle in which the scanning electromagnet moves, and as this circle in which the scanning electromagnet moves becomes larger, the device must be correspondingly increased in size.

Therefore, it is conceivable to maintain a compact device size by increasing the current value used in the scanning electromagnet above normal levels to generate a magnetic field strong enough to scan the charged particle beam. However, when using high currents, the cooling of the scanning electromagnet becomes more critical. As a result, effective cooling of the scanning electromagnet has become a significant challenge. An embodiment of the present invention provides a charged particle beam deflection device that enables a cooling solvent for cooling a scanning electromagnet to flow smoothly.

### First Embodiment

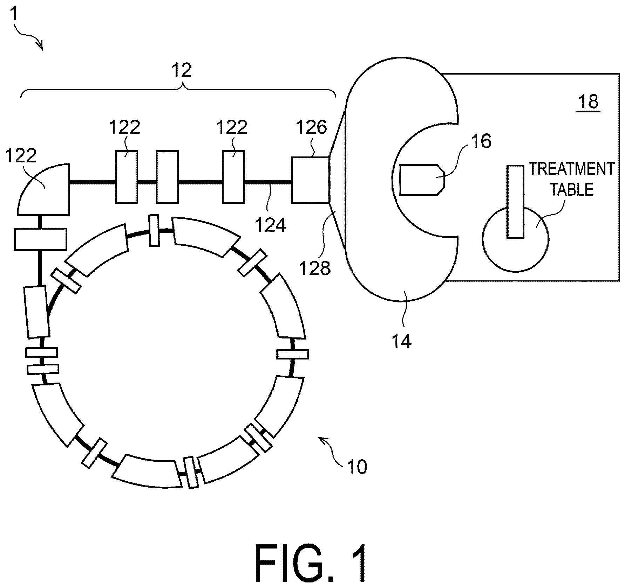

FIG. 1 is a schematic configuration view of a charged particle beam irradiation device 1 according to an embodiment of the present invention. The charged particle beam irradiation device 1 includes an accelerator 10, a charged particle beam transport system 12, a deflecting electromagnet 14, and an irradiation nozzle 16. The irradiation nozzle 16 is disposed inside a treatment room 18 provided with a treatment table on which a patient is placed.

The accelerator 10 is a device that generates a charged particle beam, and is, for example, a synchrotron, a cyclotron, or a linear accelerator. A charged particle beam generated by the accelerator 10 is guided to the deflecting electromagnet 14 through the charged particle beam transport system 12. The charged particle beam may be a beam of various known charged particles, and the charged particles may be, for example, a proton beam, alpha particles, heavy particles, or electrons.

The charged particle beam transport system 12 includes one or more charged particle beam adjustment units 122, a vacuum duct 124, a distributing electromagnet 126, a fan-shaped vacuum duct 128, and the like. The accelerator 10, the charged particle beam adjustment unit 122, and the distributing electromagnet 126 are connected by the vacuum duct 124, and the distributing electromagnet 126 and the deflecting electromagnet 14 are connected by the fan-shaped vacuum duct 128.

A charged particle beam is generated by the accelerator 10 on an upstream side, travels through the vacuum duct 124 and the fan-shaped vacuum duct 128 to avoid (or reduce) attenuation, and is guided to the deflecting electromagnet 14 on a downstream side while being adjusted by the charged particle beam adjustment unit 122.

The charged particle beam adjustment unit 122 includes a beam slit for adjusting a beam shape and/or a dose of the charged particle beam, an electromagnet for adjusting a travel direction of the charged particle beam, a quadrupole electromagnet for adjusting the beam shape of the charged particle beam, a steering electromagnet for finely adjusting a beam position of the charged particle beam, and the like as appropriate in accordance with specifications.

The irradiation nozzle 16 is located in the treatment room 18 in which treatment or the like using the charged particle beam is performed, and the charged particle beam is emitted from the irradiation nozzle 16. Treatment is performed by irradiating an affected area with the charged particle beam emitted from the irradiation nozzle 16. The irradiation nozzle 16 according to the present embodiment includes a charged particle beam deflection device for finely adjusting the travel direction of the charged particle beam emitted from the irradiation nozzle 16 by adjusting an amount of flowing current and a direction of the current, and for enabling scanning within a predetermined range. Thus, the irradiation nozzle 16 can two-dimensionally scan the charged particle beam.

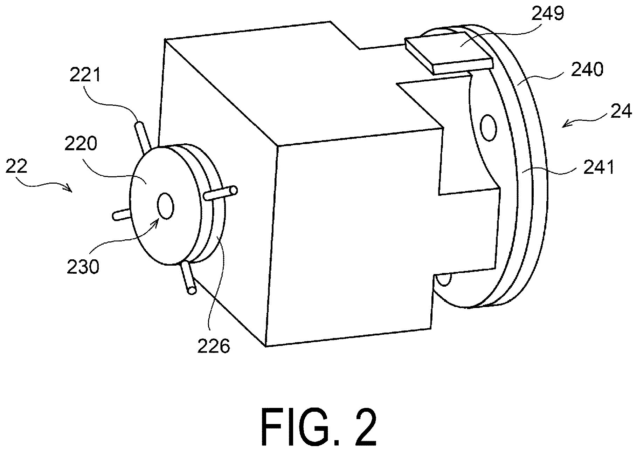

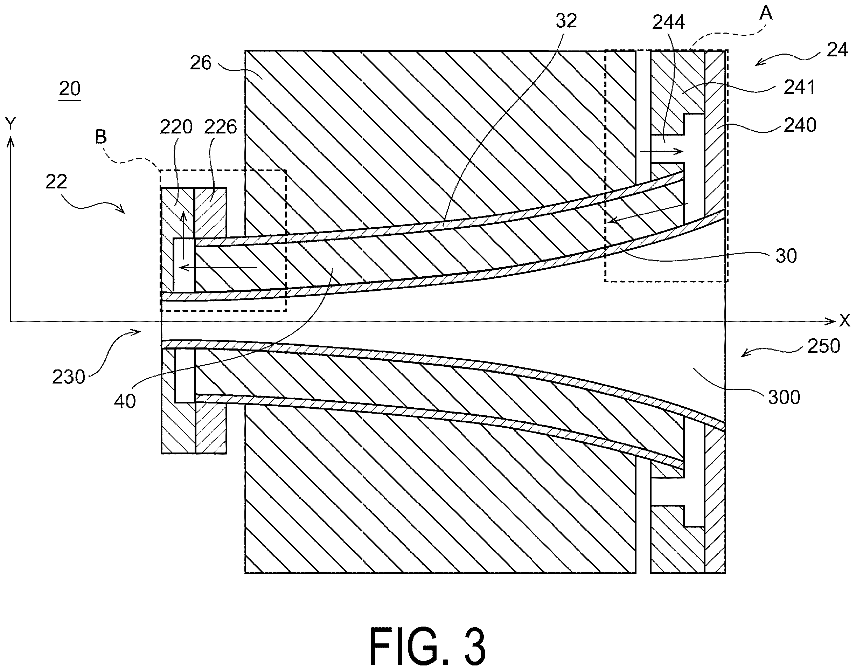

FIG. 2 is a perspective view of a charged particle beam deflection device 20 according to the present embodiment. FIG. 3 is a cross-sectional view of the charged particle beam deflection device 20 according to the present embodiment. The charged particle beam deflection device 20 according to the present embodiment includes an outflow member 22, an inflow member 24, a yoke 26, an inner tube 30 (hollow member), an outer tube 32, and a structure 40.

The inner tube 30 has a hollow shape through which the charged particle beam passes. An incident port 230 is provided at one end of the inner tube 30, and an emission port 250 is provided at the other end of the inner tube 30. The charged particle beam enters from the incident port 230, passes through an internal space 300 of the inner tube 30, and is emitted from the emission port 250. Further, the inner tube 30 is formed so that an inner diameter and an outer diameter thereof increase nonlinearly or linearly from an incident side toward an emission side.

The X-axis illustrated in FIG. 3 is an axis passing through a center axis of the inner tube 30, and a direction thereof is a direction from the incident port 230 toward the emission port 250. In the following description, the "incident side" refers to the incident port 230 side of the X-axis, and the "emission side" refers to the emission port 250 side of the X-axis. Further, a Y-axis illustrated in FIG. 3 is a direction orthogonal to the X-axis and is a radial direction of the inner tube 30. Note that, in this description, the Y-axis direction may mean a direction from an interior toward an exterior, and the opposite direction may mean a direction from the exterior toward the interior.

The structure 40 is fixed to the inner tube 30, covering an outer peripheral surface of the inner tube 30. In other words, the inner tube 30 is fixed to the structure 40 so that the outer peripheral surface thereof covers an inner peripheral surface of the structure 40. The structure 40 includes a layered structure and coils described below. The coils are each configured to function as a scanning electromagnet that deflects the charged particle beam passing through the internal space 300 of the inner tube 30. The structure 40 is formed so that an inner diameter and an outer diameter thereof increase linearly or nonlinearly from the incident side toward the emission side.

The outer tube 32 is fixed to the structure 40 so as to cover an outer peripheral surface of the structure 40. Further, the yoke 26 is fixed to the inflow member 24 by a fixing member 249 (illustrated in FIG. 2) so as to be positioned outside the outer peripheral surface of the structure 40. In the present embodiment, the yoke 26 is not provided across an entire length of the structure 40. More specifically, the yoke 26 is disposed located in a central region of the outer peripheral surface of the structure 40 and not located at an end portion on the incident side or an end portion on the emission side of the outer peripheral surface of the structure 40.

The outflow member 22 is a member fixed to the inner tube 30 and the outer tube 32 and forming part of a path through which a cooling solvent for cooling the coils included in the structure 40 passes. Further, the inflow member 24 is a member fixed to the inner tube 30 and the outer tube 32 and forming part of the path through which the cooling solvent passes. The outflow member 22 and the inflow member 24 may be constituted by, for example, an insulator such as fiber reinforced plastics (FRP). In the present embodiment, a current introduction terminal (not illustrated) for supplying a current to the coils is disposed in the vicinity of the inflow member 24, and the inflow member 24 can be insulated from the structure 40 and the current introduction terminal by the inflow member 24 being constituted by an insulator. Further, the outflow member 22 and the inflow member 24 may be constituted by combining a plurality of members with a non-magnetic material such as stainless steel (SUS). This facilitates the assembly process, and a screw process and welding, for example, can be easily performed.

The cooling solvent may be any known coolable liquid, and in the present embodiment, the cooling solvent is water. In FIG. 3, arrows illustrated in the outflow member 22, the inflow member 24, and the structure 40 indicate directions in which the cooling solvent flows. That is, the cooling solvent flows in from the inflow member 24, passes through gaps of the structure 40, and is discharged from the outflow member 22 through discharge ports 221 (illustrated in FIG. 2).

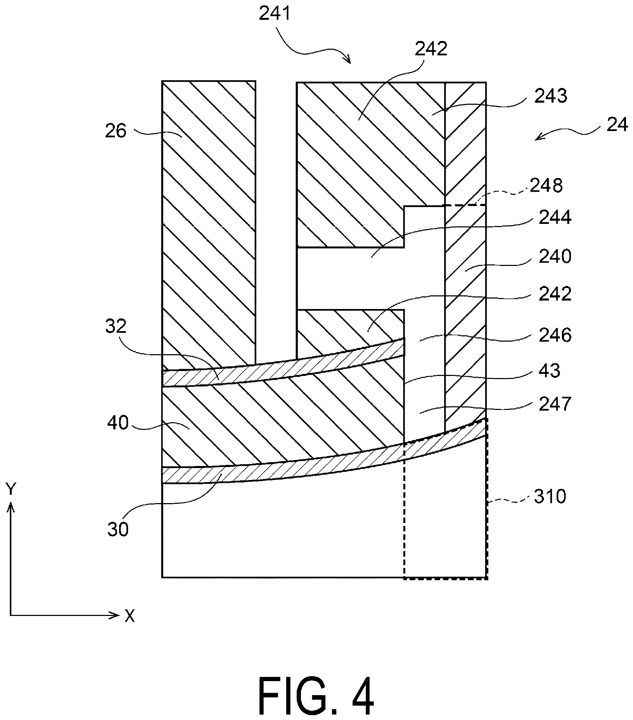

FIG. 4 is an enlarged view of a region A illustrated in FIG. 3. As illustrated in FIG. 4, the inflow member 24 includes an emission-side end portion 240 and an emission-side pressing portion 241. The emission-side end portion 240 is disposed covering an end surface 43 of the structure 40 on the emission side, and functions as a covering member. Thus, the coils and the like of the structure 40 are protected by the emission-side end portion 240. Note that a portion of the emission-side end portion 240 below a dashed line 248 may also be regarded as a covering member.

The inner tube 30 includes an inner tube protruding portion 310 protruding outward from the end surface 43 (in a direction from the incident side toward the emission side). By fixing this inner tube protruding portion 310 to another member, it is possible to further stabilize the arrangement of the inner tube 30. As a result, positions of the coils disposed in the structure 40 are more stable, making it possible to improve workability related to adjusting the control of the charged particle beam.

Further, in the present embodiment, the inner tube protruding portion 310 is fixed to the inflow member 24, and the inflow member 24 is fixed to the yoke 26 by the fixing member 249. Thus, the inner tube 30 is fixed to the yoke 26 via the inflow member 24 and a fixation device. As a result, deviation of a positional relationship between the inner tube 30 and the yoke 26 is suppressed, making the inner tube 30 more reliably disposed at an appropriate position. As a result, the coils of the structure 40 integrated with the inner tube 30 are more reliably disposed at appropriate positions.

Further, in the present embodiment, a space 246 is formed surrounded by the inner tube protruding portion 310 (specifically, an outer peripheral surface thereof), the end surface 43, and the emission-side end portion 240. In the present embodiment, the coils disposed in the structure 40 are constituted by litz wire. For example, litz wires or the like connecting a plurality of the coils disposed in the structure 40 can be collectively fixed, and the collective litz wires can be cooled by the cooling solvent by utilizing the space 246. Note that litz wire is a wire formed by twisting together a plurality of thin conductors insulated by an insulating material such as enamel.

Further, in the present embodiment, the space 246 communicates with the gaps of the structure 40 and forms part of the flow path of the cooling solvent. By utilizing the space 246 communicating with the gaps of the structure 40, it is possible to make the cooling solvent flow more smoothly with a simple structure and cool the coils of the structure 40.

The emission-side pressing portion 241 is configured to press the outer tube 32 from the exterior toward the interior. Thus, the structure 40 is pressed from the exterior toward the interior. The structure 40 according to the present embodiment has a layered structure including a plurality of layers layered in a direction from the interior toward the exterior. This layered structure is pressed by the emission-side pressing portion 241 via the outer tube 32 and thus, when the cooling solvent flows through the gaps of the layered structure, deviation of each layer constituting the layered structure is suppressed.

Further, the emission-side pressing portion 241 according to the present embodiment is an annular member located on the outer peripheral surface of the structure 40, extending around the structure 40 in a peripheral direction. Therefore, the emission-side pressing portion 241 can press the entire circumference of the structure 40, making it possible to more reliably suppress the deviation of the layers in the layered structure of the structure 40.

In the present embodiment, the emission-side pressing portion 241 includes a hollow portion 242 in which a hole 244 is formed, and an emission-side protruding portion 243 protruding further toward the emission side than the end surface 43 of the structure 40. The emission-side pressing portion 241 is coupled to the emission-side end portion 240 via the emission-side protruding portion 243. Thus, the space 246 surrounded by the end surface 43 of the structure 40, the emission-side end portion 240, and the emission-side pressing portion 241 is formed in an interior of the inflow member 24. This space 246 communicates with the gaps in the layered structure of the structure 40 and forms part of the flow path of the cooling solvent. Note that a portion of the emission-side end portion 240 above the dashed line 248 may be regarded as part of the protruding portion protruding further toward the emission side than the end surface 43 of the structure 40.

Further, the hollow portion 242 includes the hole 244 open in a direction from the end surface 43 of the structure 40 toward a center of the structure 40 and communicating with the space 246. In the present embodiment, this hole 244 forms part of the flow path of the cooling solvent. The cooling solvent flows in from the hole 244, passes through the space 246, and flows into the gaps of the structure 40.

Further, in the present embodiment, the emission-side end portion 240 is disposed away from the end surface 43 of the structure 40. The inner tube 30 functions as a closing portion that is formed between the emission-side end portion 240 included in the space 246 and the end surface 43 and closes an opening 247 on the interior side of the structure 40. Thus, the cooling solvent is suppressed from flowing in an unintended direction from the space 246.

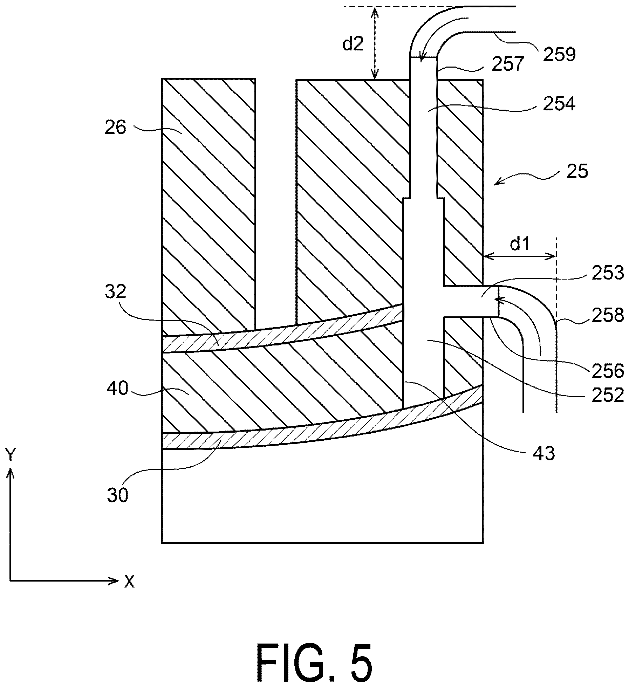

In the present embodiment, the hole 244 is open in a direction parallel to the travel direction of the charged particle beam in the emission-side pressing portion 241. With reference to FIG. 5, a case in which the hole is formed in another form will be described. In the example illustrated in FIG. 5, an inflow member 25 includes a space 252 in an interior thereof, and this space 252 communicates with a hole 253 extending from the space 252 in the X-axis direction (direction from the incident side toward the emission side) and a hole 254 extending from the space 252 in the Y-axis direction. Then, connecting portions 256, 257 for connecting hoses 258, 259 are provided at respective end portions of the hole 253 and the hole 254. The cooling solvent flows through interiors of the hoses 258, 259, the direction of which is indicated by the arrows. Note that FIG. 5 illustrates an example in which two holes, the hole 253 and the hole 254, are formed, but a case in which either one of the holes is formed is also conceivable.

As illustrated in FIG. 5, the hoses 258, 259 or the like for making the cooling solvent flow into the holes 253, 254 formed in the inflow member 25 need to be connected to the holes 253, 254. Then, a space corresponding to a width taken up by the connecting portions 256, 257 for connecting the hoses 258, 259 and the hoses 258, 259 themselves is required. For example, a space needs to be provided in the X-axis direction corresponding to a width d1 taken up by the connecting portion 256 and the hose 258 and a space needs to be provided in the Y-axis direction corresponding to a width d2 taken up by the connecting portion 257 and the hose 259. On the other hand, according to the present embodiment, in the present embodiment, the hole 244 is formed in a direction of the X-axis opposite to the hole 253 in the emission-side pressing portion 241, and thus the device is not large in the X-axis direction, the Y-axis direction, or the radial direction of the X-axis direction, making it possible to further reduce the size of the charged particle beam deflection device.

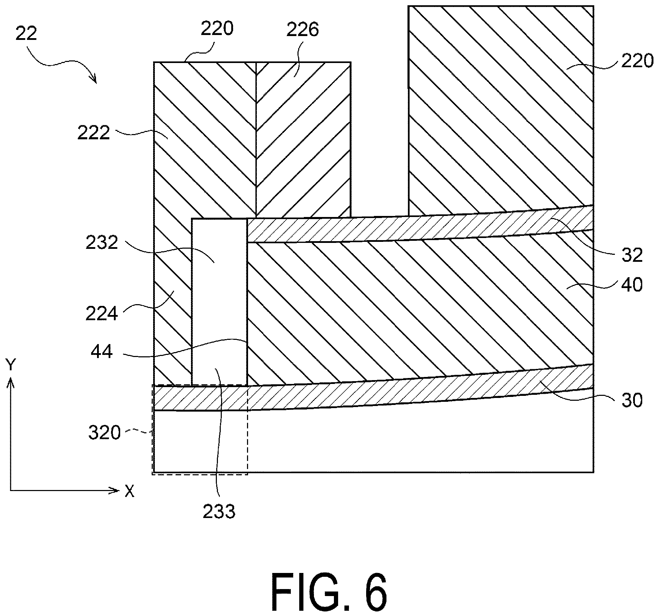

FIG. 6 is an enlarged view of a region B illustrated in FIG. 3. As illustrated in FIG. 6, the outflow member 22 includes an incident-side end portion 220 and an incident-side pressing portion 226. The incident-side end portion 220 includes an incident-side protruding portion 222 provided protruding from the incident-side pressing portion 226 in an incident-side direction and an incident-side covering portion 224 disposed covering an end surface 44 of the structure 40 on the incident side. The incident-side covering portion 224 functions as a covering member, and the coils and the like of the structure 40 are protected by the incident-side covering portion 224.

The structure 40 includes the end surface 44 on a side where the charged particle beam is incident. Further, the inner tube 30 includes an inner tube protruding portion 320 protruding in a direction outward from the end surface 44 (direction from the emission side toward the incident side). By fixing this inner tube protruding portion 320 to another member, it is possible to further stabilize the arrangement of the inner tube 30. As a result, the position of the scanning electromagnet becomes more stable, making it possible to improve the workability related to adjusting the control of the charged particle beam.

Further, in the present embodiment, a space 232 is formed surrounded by the inner tube protruding portion 320 (more specifically, an outer peripheral surface thereof), the end surface 44, and the incident-side end portion 220. For example, litz wire or the like connecting a plurality of the coils disposed in the structure 40 can be collectively fixed, and the collective litz wires can be cooled by the cooling solvent by utilizing the space 232.

Further, the space 232 communicates with the gaps of the structure 40 and forms part of the flow path of the cooling solvent. In the present embodiment, the cooling solvent can be made to flow more smoothly with a simple structure by utilizing the space 232 communicating with the gaps of the layered structure. The cooling solvent flowing from the structure 40 into the space 232 is discharged from the discharge ports 221 illustrated in FIG. 2 through a hole (not illustrated).

The incident-side pressing portion 226 is configured to press the outer tube 32 from the exterior toward the interior. The structure 40 is pressed from the exterior toward the interior by the incident-side pressing portion 226. Therefore, when the cooling solvent flows through the structure 40, deviation of each layer constituting the layered structure of the structure 40 is suppressed.

Further, in the present embodiment, the incident-side pressing portion 226 is an annular member located on the outer peripheral surface of the structure 40, extending around the structure 40 in the peripheral direction. Therefore, the incident-side pressing portion 226 can press the entire circumference of the structure 40, making it possible to more reliably suppress the deviation of the layers in the layered structure of the structure 40.

In the present embodiment, the incident-side end portion 220 is coupled to the incident-side pressing portion 226 through the incident-side protruding portion 222. Thus, the space 232 surrounded by the incident-side end portion 220 and the end surface 44 of the structure 40 is formed in the interior of the outflow member 22. This space 232 communicates with the gaps in the layered structure of the structure 40 and forms part of the flow path of the cooling solvent.

Further, the incident-side covering portion 224 is disposed away from the end surface 44 of the structure 40, forming an opening 233 between the incident-side covering portion 224 included in the space 232 and the end surface 44. In the present embodiment, the inner tube 30 functions as a closing portion that closes the opening 233 on the interior side of the structure 40. Thus, the flow of the cooling solvent flowing in the space 232 in an unintended direction is suppressed.

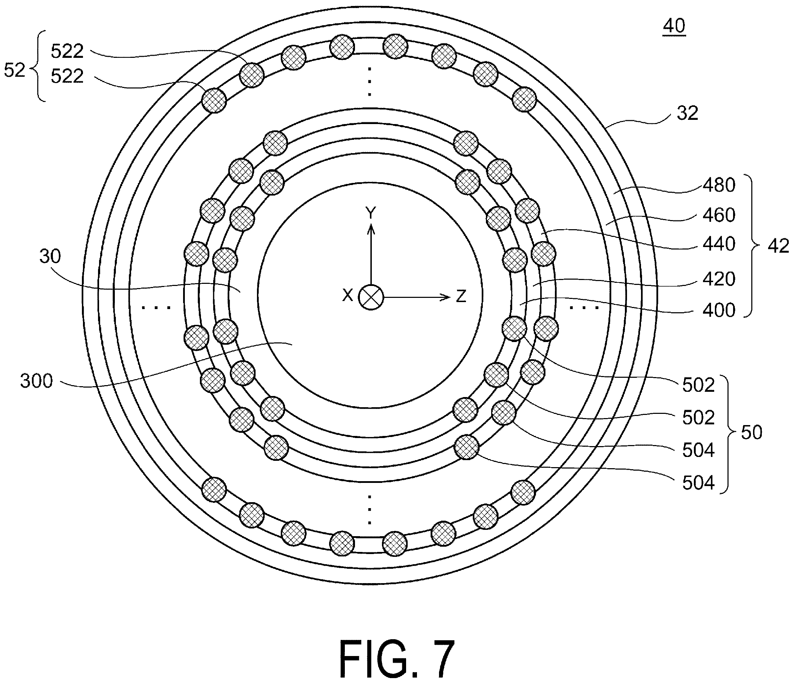

FIG. 7 is a view of a cross section orthogonal to the X-axis direction of the structure 40. In FIG. 7, a Z-axis is an axis orthogonal to the X-axis and the Y-axis. The structure 40 according to the present embodiment includes a layered structure 42, first coils 50 that deflect the charged particle beam in a first direction different from the travel direction of the charged particle beam, and second coils 52 that deflect the charged particle beam in a second direction different from the travel direction of the charged particle beam and the first direction.

The first coils 50 and the second coils 52 are each disposed forming pairs facing each other across the internal space 300. Each pair of the first coils 50 and the second coils 52 may be disposed in parallel. That is, as illustrated in FIG. 7, the first coils 50 and the second coils 52 may be disposed so that there exists a cross section of the structure 40 orthogonal to the X-axis that includes both coils. When a current flows through the first coils 50 and the second coils 52, the first coils 50 and the second coils 52 generate a magnetic field, and the charged particle beam is deflected by this magnetic field.

The layered structure 42 covers the outer peripheral surface of the inner tube 30 and includes a plurality of layers layered in a direction from an interior toward an exterior of the inner tube 30. In other words, an inner peripheral surface of the layered structure 42 is covered with the outer peripheral surface of the inner tube 30. Each of the plurality of layers included in the layered structure 42 may be made of, for example, various synthetic resins. Further, the layered structure 42 includes a gap between two adjacent layers through which the cooling solvent can flow. Each of these layers has a hollow shape, and is formed so that an inner diameter and an outer diameter thereof increase linearly or nonlinearly from the incident side toward the emission side.

The layered structure 42 according to the present embodiment includes a plurality of winding frame layers (coil support layers) on which the coils are disposed, and a plurality of water channel layers disposed between two adjacent winding frame layers and forming a path of the cooling solvent. In the present embodiment, the coils disposed on two adjacent winding frame layers are connected to each other. The first coils 50 disposed on the respective winding frame layers are electrically connected to each other and, similarly, the second coils 52 disposed on the respective winding frame layers are electrically connected to each other.

The structure 40 according to the present embodiment includes nine winding frame layers and nine water channel layers. More specifically, the structure 40 includes a first winding frame layer 400, a first water channel layer 420, a second winding frame layer 440, ..., a ninth winding frame layer 460, and a ninth water channel layer 480 in this order from the inside. Although the structure 40 according to the present embodiment includes nine winding frame layers, FIG. 7 illustrates two water channel layers among the nine winding frame layers. Note that the number of winding frame layers may be eight layers or less or may be ten layers or more. Further, although the layered structure 42 according to the present embodiment includes nine water channel layers, FIG. 7 illustrates three winding frame layers among the nine water channel layers. Note that the number of water channel layers may be eight layers or less or may be ten layers or more.

The first winding frame layer 400 is disposed covering the outer peripheral surface of the inner tube 30, and is a coil support layer (first coil support layer) that supports coils 502 (first coils) that deflect the charged particle beam. The coils 502 according to the present embodiment generate a magnetic field in the Z-axis direction and deflect the charged particle beam by this magnetic field.

The first water channel layer 420 is disposed covering an outer peripheral surface of the first winding frame layer 400, and is a layer that forms part of the path through which the cooling solvent passes.

The second winding frame layer 440 is a layer in which coils 504 (first coils) that deflect the charged particle beam are disposed. As with the coils 502, the coils 504 generate a magnetic field in the Z-axis direction and deflect the charged particle beam by this magnetic field.

Although not illustrated in FIG. 7, the third to eighth winding frame layers and the second to eighth water channel layers are layered on an outer peripheral surface of the second winding frame layer 440 with the winding frame layers and the water channel layers alternately overlapping. Each of the third to fifth winding frame layers is a coil support layer (first coil support layer) that supports the coils (first coils), and each coil is configured to generate a magnetic field in the Z-axis direction, similarly to the coils 502, 504.

The ninth winding frame layer 460 is disposed covering an outer peripheral surface of the eighth water channel layer (not illustrated), and is a coil support layer (second coil support layer) that supports coils 522 (second coils) that deflect the charged particle beam in a direction (second direction) different from the direction (first direction) in which the coils disposed on the first to fifth winding frames (coils 502, 504, for example) deflect the charged particle beam.

The coils 522 are disposed on the ninth winding frame layer 460. The coils 522 generate a magnetic field in the Y-axis direction and deflect the charged particle beam traveling in the X-axis direction in the Z-axis direction. Further, the coils (second coils) are disposed in each of the sixth to eighth winding frame layers described above, and each coil is configured to generate a magnetic field in the Y-axis direction, similarly to the coils 522.

The ninth water channel layer 480 is disposed covering an outer peripheral surface of the ninth winding frame layer 460, and is a layer that forms part of the path through which the cooling solvent passes. The outer tube 32 is disposed on an outer peripheral surface of the ninth water channel layer 480. The outer tube 32 is disposed so that an inner peripheral surface thereof covers the outer peripheral surface of the ninth water channel layer 480.

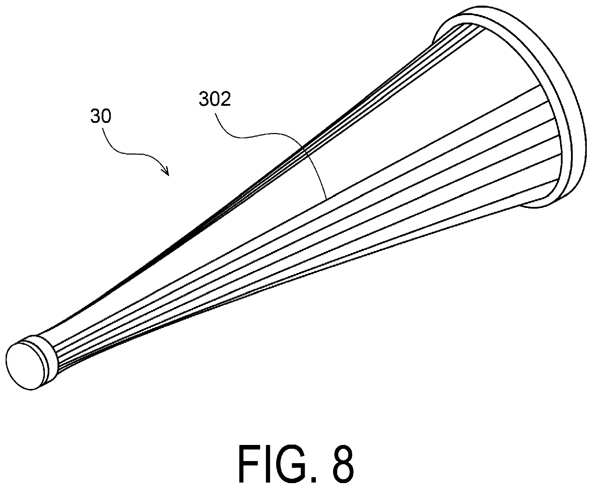

FIG. 8 is a perspective view of the inner tube 30 according to the present embodiment. As illustrated in FIG. 8, grooves 302 are formed on the outer peripheral surface of the inner tube 30 along a longitudinal direction. These grooves 302 form part of the path of the cooling solvent.

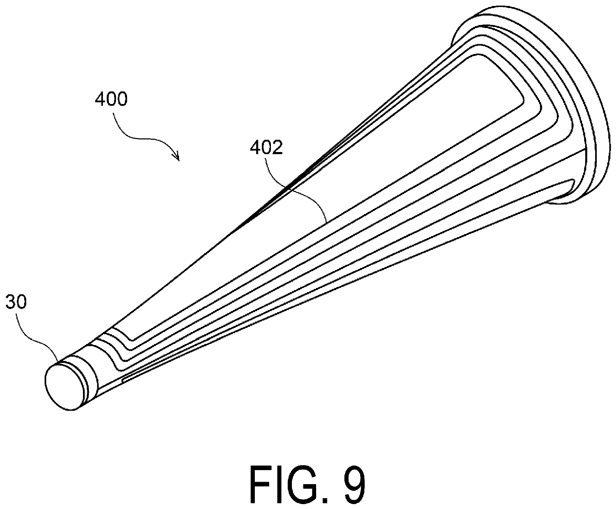

FIG. 9 is a perspective view of the first winding frame layer 400 disposed on the inner tube 30. Grooves 402 are formed in the first winding frame layer 400. Litz wire is embedded in these grooves 402 and solidified by a resin, whereby the litz wire constitutes the coils (first coils).

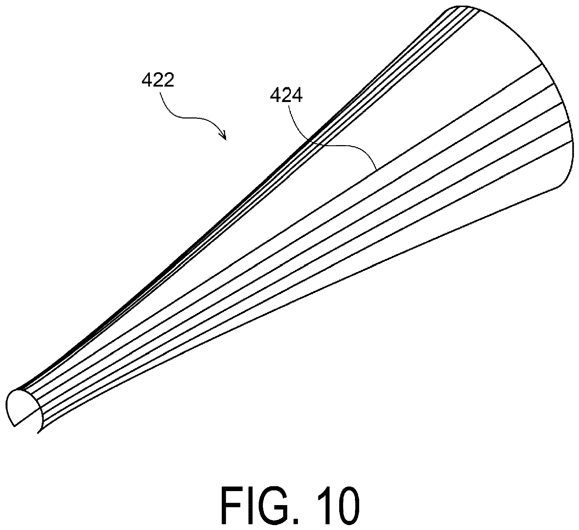

FIG. 10 is a perspective view illustrating a water channel member 422 of the first water channel layer 420 according to the present embodiment. The water channel member 422 illustrated in FIG. 10 constitutes a half circumference of the first water channel layer 420. There is another water channel member having the same configuration as that of the water channel member 422 illustrated in FIG. 10, and the two water channel members are combined, thereby forming the first water channel layer 420 extending around the outer peripheral surface of the first winding frame layer 400. The water channel member 422 includes the grooves 424 on an outer peripheral surface thereof, and these grooves 424 form part of a path through which the cooling solvent passes. Note that, as with the first water channel layer, for each water channel layer included in the layered structure 42, two water channel members are combined, thereby forming the water channel layer extending around the circumference. In the present embodiment, an example in which each water channel layer is formed of water channel members will be described. However, each water channel layer may be formed as a simple space without the use of a water channel member.

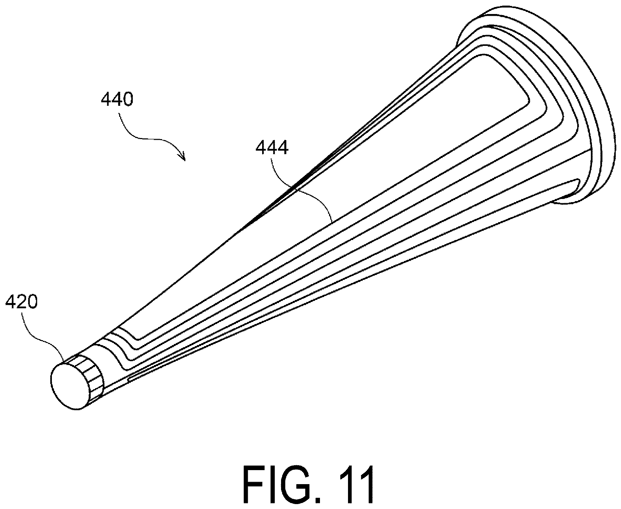

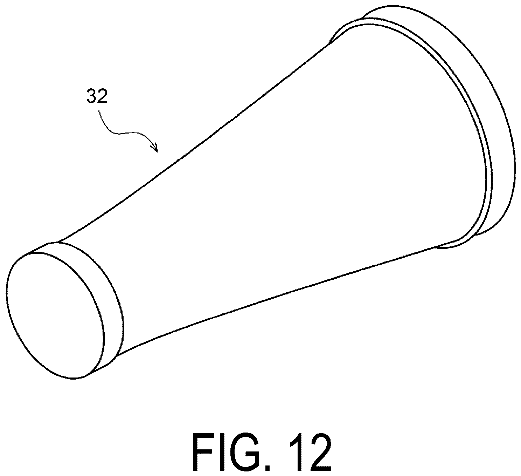

FIG. 11 is a perspective view of the second winding frame layer 440 according to the present embodiment. FIG. 11 illustrates a state in which the second winding frame layer 440 is disposed on a surface of the first water channel layer 420. Grooves 444 are formed on the surface of the second winding frame layer 440, litz wire is embedded in the grooves 444, and the litz wire constitutes the coils 504. The grooves 444 may each be formed as a hole penetrating the second winding frame layer 440 from a front surface to a back surface, or may be formed so as to not penetrate the second winding frame layer 440 from the front surface to the back surface. Further, the second water channel layer is disposed on an outer surface of the second winding frame layer 440, covering the outer surface. Grooves are provided on an inner peripheral surface of the second water channel layer, and part of the path through which the cooling solvent passes is formed by these grooves and the outer surface of the second winding frame layer 440. In this way, the winding frame layers and the water channel layers are layered on the outer peripheral surface of the inner tube 30, and the outer tube 32 illustrated in FIG. 12 is disposed on an outer peripheral surface of the outermost water channel layer, covering the outer peripheral surface.

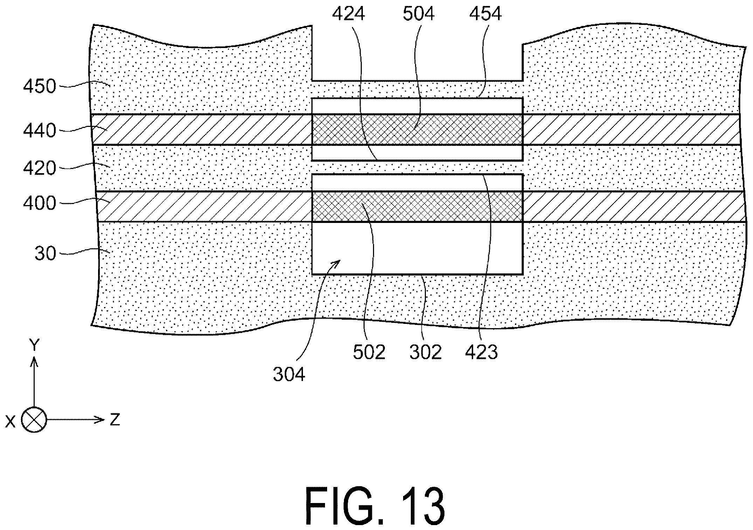

FIG. 13 is an enlarged view illustrating part of a cross section of the inner tube 30 and the structure 40, the cross section being orthogonal to the X-axis. In the present embodiment, the inner tube 30 is disposed forming a gap 304 between the inner peripheral surface of the layered structure 42 and the outer peripheral surface of the inner tube 30 so that the outer peripheral surface of the inner tube 30 does not come into contact with the first coils and the second coils. Specifically, a path through which the cooling solvent flows is formed between the grooves 302 provided on the outer surface of the inner tube 30 and the coils 502 disposed on the first winding frame layer 400 so that the grooves 302 of the inner tube 30 do not come into contact with the coils 502. Therefore, as compared with a case in which the inner tube 30 comes into contact with the coils 502, in the charged particle beam deflection device according to the present embodiment, the cooling solvent can be made to flow through the gap where the inner tube 30 and the coils 502 do not come into contact with each other, making it possible to cool the coils 502 more efficiently with the cooling solvent.

Further, a path through which the cooling solvent flows is formed between grooves 423 formed on an inner surface of the first water channel layer 420 and the coils 502, and between grooves 424 formed on an outer surface of the first water channel layer 420 and the coils 504 disposed on the second winding frame layer 440. Furthermore, a path through which the cooling solvent flows is formed between the grooves 454 formed on an inner surface of a second water channel layer 450 and the coils 504. Similarly, the grooves formed in an outer surface of the second water channel layer 450 and the grooves formed in the surface of each water channel layer form paths through which the cooling solvent flows between the winding frame layers or the coils adjacent to each other. By making the cooling solvent flow through these paths, the coils can be cooled more efficiently.

In the treatment using the charged particle beam, the coils need to be arranged at desired positions with high precision in order to increase the precision of the position where the charged particle beam is emitted. In the charged particle beam deflection device 20 according to the present embodiment, the layered structure 42 in which the coils are disposed is fixed to the inner tube 30. Therefore, as long as the inner tube 30 is disposed at an appropriate position, the coils are naturally disposed at appropriate positions by appropriately installing the layered structure 42 with respect to the inner tube 30. In other words, the layered structure 42 and the inner tube 30 are integrated with each other, making it possible to install the coils at appropriate positions by adjusting the position of the inner tube 30. Thus, in the present embodiment, by fixing the layered structure 42 to the inner tube 30, it is possible to improve the precision of the position of the scanning electromagnet. As a result, it is possible to make the work for adjusting the irradiation position of the charged particle beam more efficient.

Further, as described above, in the charged particle beam deflection device 20 according to the present embodiment, the inner tube 30 (hollow member) is disposed so that a gap in which an outer peripheral surface 332 of the inner tube 30 does not come into contact with the first coils and the second coils is formed between the inner peripheral surface of the layered structure 42 and the outer peripheral surface 332 of the inner tube 30. Therefore, in a gap 306 between the inner peripheral surface of the layered structure 42 and the outer peripheral surface 332 of the inner tube 30, the inner tube 30 does not come into direct contact with the litz wire constituting the first coils or the second coils. Therefore, as compared with a case in which the inner tube 30 comes into contact with the coils in the gap, an area in which the coils can come into contact with the cooling solvent is increased. As a result, the coils disposed in the layered structure 42 (particularly, the coils 502 disposed in the first winding frame layer 400) can be more efficiently cooled by the cooling solvent.

Further, in the present embodiment, grooves forming part of the flow path of the cooling solvent are provided on the outer peripheral surface of the inner tube 30. Therefore, in the present embodiment, the cooling solvent can be brought into direct contact with the innermost coils, making it possible to cool the coils more efficiently.

As described above, in the charged particle beam deflection device according to the present embodiment, a large current flows through the coils. The coils therefore need to be cooled more reliably by the cooling solvent. As the cooling solvent flows through the gaps between the layers of the layered structure, the layers may deviate in position. When the layers deviate in position, the flow of the cooling solvent may be impeded, and cooling may not be performed smoothly. When the cooling is not performed smoothly, the coils may become very hot due to the high current.

In the present embodiment, the incident-side pressing portion 226 and the emission-side pressing portion 241 press the layered structure 42 from the exterior toward the interior. Thus, the layers included in the layered structure 42 are pressed by these pressing portions, suppressing deviation in the positions of the layers. This makes it possible to make the cooling solvent flow into the gaps of the layered structure 42 without interruption and smoothly cool the coils.

Further, in the present embodiment, the outflow member 22, the inflow member 24, the inner tube 30, and the structure 40 form the spaces 232, 246 communicating with the gaps of the structure 40, and these spaces are used as water channels through which the cooling solvent flows. This makes it possible to realize the water channel with a simple structure, and make the cooling solvent flow smoothly. Further, litz wire or the like connecting the layers of the winding frame layers can be collectively fixed in these spaces. In this case, the litz wire can be collectively cooled.

Further, according to the present embodiment, because deviation of each layer in the layered structure 42 can be suppressed, it is possible to suppress deviation in the positions of the coils disposed in the winding frame layers. This makes it possible to make the coils generate magnetic fields as designed with higher precision.

### First Modification

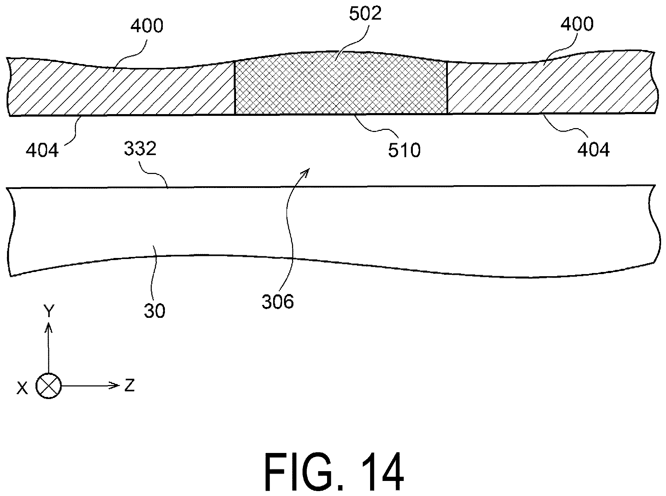

FIG. 14 is an enlarged view illustrating part of a cross section of the inner tube 30 and the structure 40 according to a first modification, the cross section being orthogonal to the X-axis. In the example illustrated in FIG. 14, it is assumed that the cooling solvent flows in the X-axis direction (direction from the back side to the front side of the paper surface) in the gap formed between the inner tube 30 and the first winding frame layer 400 and the coils 502.

In the first modification, as illustrated in FIG. 14, the inner tube 30 is disposed forming a gap in which contact is not made with the coils 502 disposed in the first winding frame layer 400. More specifically, the inner tube 30 is disposed forming the gap 306 in which the outer peripheral surface 332 thereof does not come into contact with an inner surface 404 of the first winding frame layer 400 and inner surfaces 510 of the coils 502. In the first modification, the gap 306 is formed so that the cooling solvent comes into contact with the first winding frame layer 400. Even in such a form, the coils 502 can be efficiently cooled by the cooling solvent.

Thus, in the charged particle beam deflection device according to the present embodiment, as compared with a case in which the inner tube 30 comes into contact with the coils 502, the cooling solvent can be made to flow through the gap where contact is not made, making it possible to cool the coils 502 more efficiently with the cooling solvent.

### Second Modification

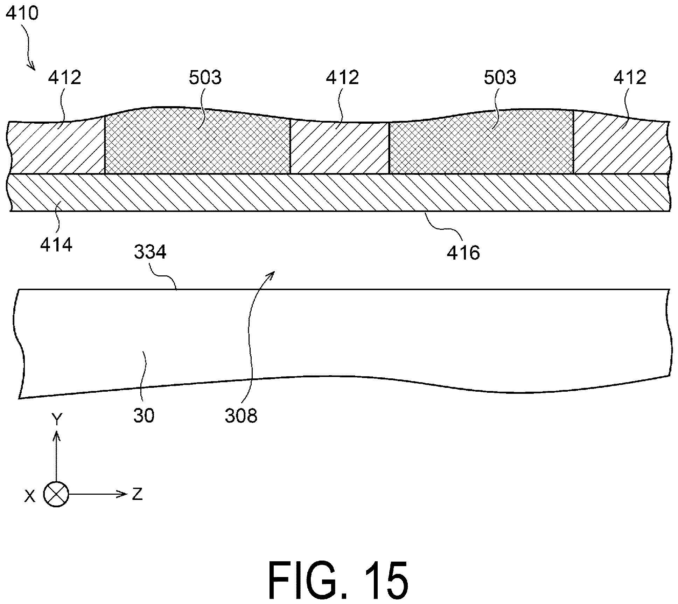

FIG. 15 is an enlarged view illustrating part of a cross section of the inner tube 30 and the structure 40 according to a modification, the cross section being orthogonal to the X-axis. In a second modification, a first winding frame layer 410 includes a support portion 412 that sandwiches and supports coils 503 and, in addition thereto, a bottom portion 414 that covers the inner tube 30 side of the coils 503. In the second modification, it is assumed that the cooling solvent flows in the X-axis direction (direction from the back side to the front side of the paper surface) in a gap formed between the inner tube 30 and the bottom portion 414. In the second modification, the coils 503 are cooled by the cooling solvent through the bottom portion 414. In the second modification as well, the inner tube 30 is disposed so as to form a gap in which contact is not made with the coils 503. The cooling solvent can therefore be made to flow through the gap where the inner tube 30 and the coils 503 do not come into contact with each other, making it possible to cool the coils 503 more efficiently with the cooling solvent.

The inner tube 30 is disposed forming a gap 308 between the inner peripheral surface of the layered structure (more specifically, a bottom surface 416 of the bottom portion 414) and an outer peripheral surface 334 of the inner tube 30 so that the outer peripheral surface 334 of the inner tube 30 does not come into contact with the coils 503. This allows the cooling solvent to flow through the gap where the inner tube 30 and the coils do not come into contact with each other, making it possible to cool the coils 503 more efficiently with the cooling solvent.

### Second Embodiment

The first embodiment described an example in which the first coils and the second coils are disposed in parallel. However, the configuration is not limited thereto, and the first coils and the second coils may be disposed in series. That is, the first coils and the second coils may be disposed at positions shifted from each other in a direction parallel to the X-axis.

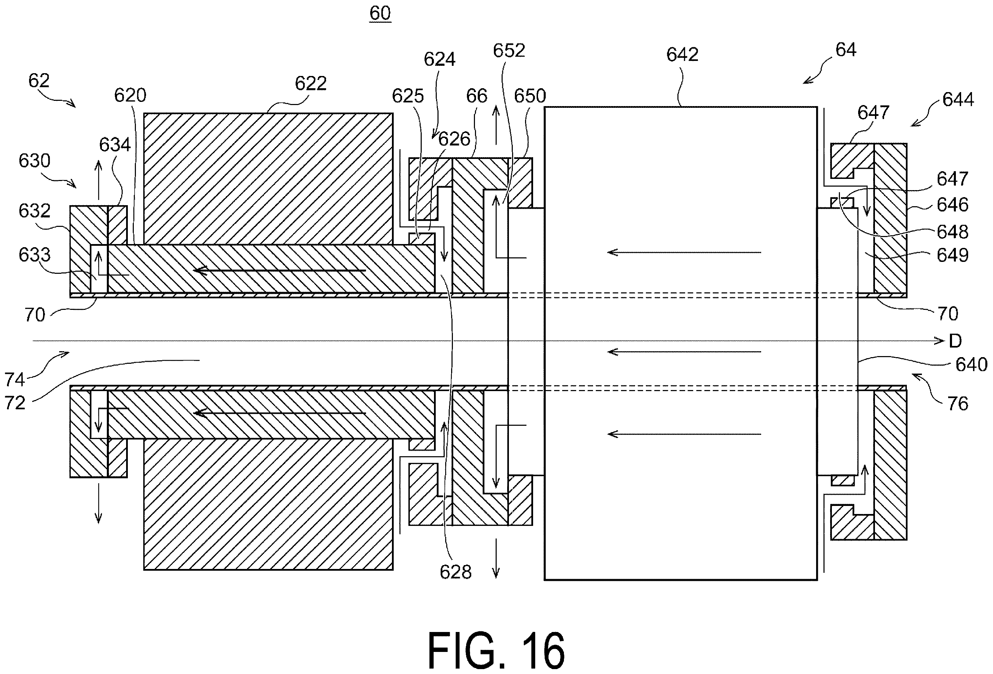

With reference to FIG. 16, a charged particle beam deflection device 60 according to a second embodiment in which the first coils and the second coils are disposed in series will now be described. The charged particle beam deflection device 60 according to the second embodiment mainly includes a first deflection device 62, a second deflection device 64, and a coupling member 66 coupling the first deflection device 62 and the second deflection device 64. Each of the first deflection device 62 and the second deflection device 64 may include various configurations included in the charged particle beam deflection device 20 according to the first embodiment.

An internal space 72 including an incident port 74 at one end and an emission port 76 at the other end is formed in an interior of the charged particle beam deflection device 60. A charged particle beam is incident on the internal space 72 from the incident port 74 in a D-axis direction illustrated in FIG. 16, is deflected by the first deflection device 62 and the second deflection device 64 in the internal space 72, and is emitted from the emission port 76.

The first deflection device 62 is a device that deflects the charged particle beam in a direction orthogonal to the D-axis direction and causes the deflected charged particle beam to be incident on the second deflection device 64. The first deflection device 62 includes a first structure 620, a first yoke 622, a first inflow member 624, a first outflow member 630, and an inner tube 70.

The inner tube 70 has a hollow shape and is configured so that the charged particle beam passes through an interior thereof. In the present embodiment, the inner tube 70 is shared by the first deflection device 62 and the second deflection device 64. However, the configuration is not limited thereto, and the first deflection device 62 and the second deflection device 64 may be independently formed. The incident port 74 is formed at one end of the inner tube 70 and the emission port 76 is formed at the other end of the inner tube 70. Further, the inner tube 70 according to the second embodiment has a cylindrical shape. Note that an outer diameter and an inner diameter thereof may have a linear or non-linear slope.

The first structure 620 is configured to deflect the charged particle beam passing through the internal space 72 by a magnetic field. Although not illustrated, the first structure 620 includes a first layered structure and first coils. The first coils deflect the charged particle beam in a direction different from the D-axis direction by the generated magnetic field. For example, the first coils may deflect the charged particle beam in a direction orthogonal to the D-axis direction.

The first layered structure has a hollow shape through which the charged particle beam passes, and includes a plurality of layers layered in a direction from the interior toward the exterior. Between two adjacent layers among the plurality of layers, there is a gap that allows the cooling solvent to flow therethrough. Further, the plurality of layers included in the first layered structure include a first coil support layer that supports the first coils.

The first inflow member 624 is a member for allowing the cooling solvent to flow into the first structure 620. The first inflow member 624 is connected to the coupling member 66. The first inflow member 624 includes a pressing portion 625 that presses the first structure 620 from the outside toward the inside. Further, a space 628 surrounded by an end surface of the first structure 620, the first inflow member 624, the coupling member 66, and an outer peripheral surface of the inner tube 70 is formed in an interior of the first inflow member 624, and the space 628 communicates with the gap of the first structure 620. Furthermore, the first inflow member 624 includes a hole 626 open in the D-axis direction, and the hole 626 communicates with the space 628.

The first outflow member 630 is a member for making the cooling solvent flow to the exterior. The first outflow member 630 includes a pressing portion 634 that presses the first structure 620 from the exterior toward the interior, and a covering member 632 that covers the end surface of the first structure 620. In the second embodiment, a space 633 is formed surrounded by the end surface of the first structure 620, the covering member 632, and the outer peripheral surface of the inner tube 70. The space 633 communicates with the gap of the first structure 620 and the discharge port (not illustrated).

In the first deflection device 62, the cooling solvent flows through the first inflow member 624, the first structure 620, and the first outflow member 630 in the direction of the arrow illustrated in FIG. 16, thereby cooling the first coils included in the first structure 620. Specifically, the cooling solvent flows from the hole 626 of the first inflow member 624 into the space 628, passes through the first structure 620 and the space 633 of the first outflow member 630, and is discharged from the discharge port. At this time, the first coils are cooled by the cooling solvent flowing through the gap of the first structure 620.

The second deflection device 64 is a device that deflects the charged particle beam in a direction different from the D-axis direction and the direction in which the charged particle beam is deflected by the first deflection device 62, and causes the deflected charged particle beam to be emitted from the emission port 76. The second deflection device 64 includes a second structure 640, a second yoke 642, a second inflow member 644, a second outflow member 650, and the inner tube 70.

The second structure 640 is configured to deflect the charged particle beam passing through the internal space 72 by a magnetic field. Although not illustrated, the second structure 640 according to the second embodiment includes a second layered structure and second coils. The second coils deflect the charged particle beam deflected by the first deflection device 62 in a direction different from the D-axis direction and the direction in which the charged particle beam is deflected by the first deflection device 62 by the generated magnetic field. The second coils may deflect the charged particle beam, for example, in a direction orthogonal to the D-axis direction and orthogonal to the direction in which the first deflection device 62 deflects the charged particle beam.

The second layered structure has a hollow shape through which the charged particle beam passes, and includes a plurality of layers layered in a direction from the interior toward the exterior. Between two adjacent layers among the plurality of layers, there is a gap that allows the cooling solvent to flow therethrough. Further, the plurality of layers included in the second layered structure include a second coil support layer that supports the second coils.

The second inflow member 644 is a member for allowing the cooling solvent to flow into the second structure 640. The second inflow member 644 includes a covering member 646 that covers an end surface of the second structure 640 and a pressing portion 647 that presses the second structure 640 from the exterior toward the interior. In the second embodiment, a space 649 is formed surrounded by the end surface of the second structure 640, the covering member 646, and the outer peripheral surface of the inner tube 70. The space 649 communicates with the gap of the second structure 640. Further, the second inflow member 644 includes a hole 648 open in the D-axis direction, and the hole 648 communicates with the space 649.

The second outflow member 650 is a member for making the cooling solvent flow out to the exterior. The second outflow member 650 is connected to the coupling member 66. Further, the second outflow member 650 may function as a pressing portion that presses the second structure 640 from the outside toward the inside. Further, a space 652 is formed surrounded by the end surface of the second structure 640, the second outflow member 650, the coupling member 66, and the outer peripheral surface of the inner tube 70. This space 652 communicates with the gap of the second structure 640 and a discharge port (not illustrated).

In the second deflection device 64, the cooling solvent flows through the second inflow member 644, the second structure 640, and the second outflow member 650 in the direction of the arrow illustrated in FIG. 16, thereby cooling the second coils included in the second structure 640. Specifically, the cooling solvent flows from the hole 648 of the second inflow member 644 into the space 649, and is discharged from the discharge port via the second structure 640 and the space 652. At this time, the second coils are cooled by the cooling solvent flowing through the gap of the second structure 640.

The charged particle beam deflection device 60 according to the second embodiment has been described above. The second embodiment describes an example in which the first yoke 622 included in the first deflection device 62 and the second yoke 642 included in the second deflection device 64 are separate bodies. However, the configuration is not limited thereto, and the yokes included in the first deflection device 62 and the second deflection device 64 may be of an integrated type in which the two are coupled to each other.

Further, the path through which the cooling solvent flows is not limited to that in the example described above. For example, a hole enabling communication between the space 628 and the space 652 may be provided in the coupling member 66. In this case, the cooling solvent flowing into the second deflection device 64 passes through the hole provided in the coupling member 66, flows to the first deflection device 62, and is discharged from the space 633 to the exterior.

Further, the two spaces formed by the coupling member 66 (that is, the space 628 of the first deflection device 62 and the space 652 of the second deflection device 64) may both be utilized for inflow of the cooling solvent. In this case, the cooling solvent having flowed into the space 652 of the second deflection device 64 may pass through the space 649 and be discharged to the exterior from the hole 648.

### Positioning of Layered Structure

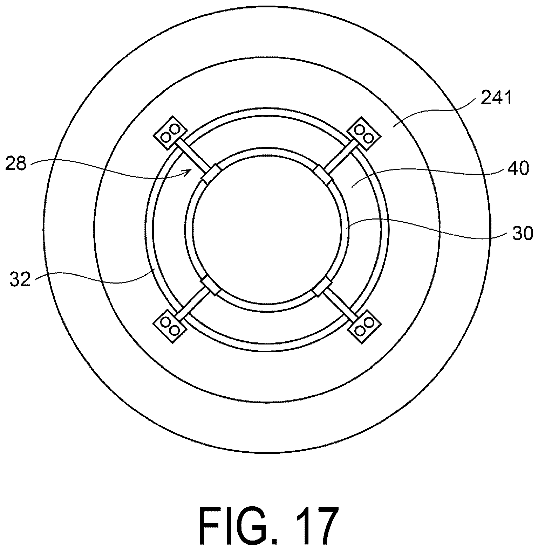

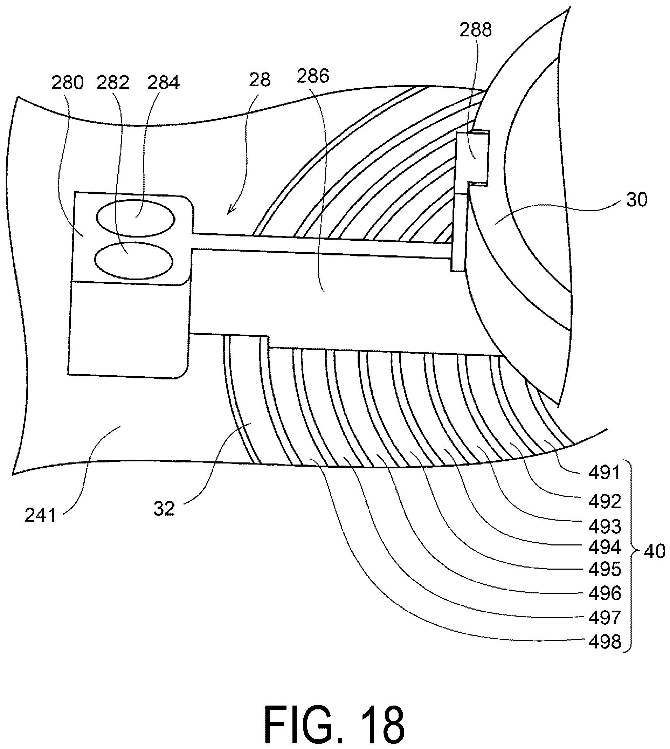

When the structure 40 is positioned with respect to the isocenter, the inflow member 24 can be set as a reference point for positioning the structure 40. This makes it possible to position the structure 40 with reference to the inflow member 24. An example of a method for positioning the structure 40 will be described below with reference to FIGS. 17 and 18. FIG. 17 is a view of the structure 40 positioned as viewed from the emission side of the charged particle beam. FIG. 18 is an enlarged view illustrating a state in which the inner tube 30, the outer tube 32, and the structure 40 are positioned with respect to the emission-side pressing portion 241 by positioning pins 28.

As illustrated in FIG. 17, the structure 40 and the inner tube 30 are fixed to the emission-side pressing portion 241 by four positioning pins 28. Note that the number of the positioning pins 28 may be three or less, or may be five or more.

Layers 491 to 498 of the structure 40 are each provided with a cutout portion formed in the radial direction. The inner tube 30 includes screw holes for fixing the positioning pins 28 to the inner tube 30. As illustrated in FIG. 18, the positioning pins 28 are engaged with the cutout portions, and the cutout portions and the screw holes of the inner tube 30 are all aligned in the radial direction, whereby each layer (each winding frame layer and each water channel layer) of the structure 40 is layered with high precision.

To engage the positioning pins 28 with the structure 40, the inner tube 30, the structure 40, and the outer tube 32 in close contact with the structure 40 are positioned relative to the emission-side pressing portion 241. Further, in a case in which a plurality of the winding frame layers provided with coils are layered as in the present embodiment, an appropriate relative positional relationship between the first coils and the second coils is ensured by the arrangement of the positioning pins 28.

With reference to FIG. 18, a configuration of the positioning pins 28 will now be described in more detail. As illustrated in FIG. 18, the positioning pins 28 each include a first to-be-fixed portion 280, a fixing portion 286, and a second to-be-fixed portion 288. The first to-be-fixed portion 280, the fixing portion 286, and the second to-be-fixed portion 288 are integrally formed.

The first to-be-fixed portion 280 includes screw holes 282, 284, and screws are inserted into the screw holes 282, 284, thereby fixing the first to-be-fixed portion 280 to a desired position of the emission-side pressing portion 241. Further, the second to-be-fixed portion 288 includes a screw hole (not illustrated), and a screw is inserted into the screw hole, thereby fixing the second to-be-fixed portion 288 to the inner tube 30. The fixing portion 286 is a plate-like member that connects the first to-be-fixed portion 280 and the second to-be-fixed portion 288.

Each of the layers 491 to 498 of the structure 40 is provided with the cutout portion, and the fixing portion 286 is engaged with the cutout portion. With the structure 40 engaged with the four positioning pins 28 in this way, the structure 40 and the outer tube 32 in close contact with the structure 40 are positioned relative to the emission-side pressing portion 241. Using the positioning pins 28 in this way facilitates assembly in which the layered structure 42 is layered with high positional precision.

An example of a flow of positioning the outer tube 32, the structure 40, the emission-side pressing portion 241, and the like will now be described. Here, an example using a simulated member simulating the inflow member 24 and simulated member positioning pins simulating the positioning pins 28 will be described.

First, the inner tube 30 is engaged with and fixed to the simulated member positioning pins fixed to the simulated member by simulated second to-be-fixed portions (portions corresponding to the second to-be-fixed portion 288 of the simulated member positioning pins) and simulated screws. Subsequently, each layer (each winding frame layer and each water channel layer) of the structure 40 is sequentially fixed or engaged layer by layer, bonding and fixing the layers. After the structure 40 is thus positioned with respect to the simulated member, the simulated member positioning pins are removed from the simulated member. Subsequently, the simulated member is replaced with the inflow member 24 and, by the positioning pins 28, the inner tube 30 is coupled to the inflow member 24 by the second to-be-fixed portions 288 and the screws passing through the screw holes, making it possible to easily fix the structure 40 and the outer tube 32 to the emission-side pressing portion 241 along the cutout portions. Thus, the structure 40 and the like are disposed with high precision at appropriate positions with respect to the inflow member 24. Accordingly, the coils of the structure 40 are fixed in appropriate positions relative to the inflow member 24. By performing assembly in this manner, it is possible to easily and reliably arrange each layer relative to the inflow member 24 with high positional precision.

### Supplement

The present invention has been described above on the basis of the embodiments. It will be understood by those skilled in the art that the embodiments are illustrative, that various modifications can be made to the combinations of the components and processes, and that such modifications are also within the scope of the present invention.

In the embodiments described above, an example is described in which the charged particle beam deflection device 20 is provided in the irradiation nozzle 16. However, the charged particle beam deflection device may be provided in various devices for deflecting a charged particle beam, such as the charged particle beam adjustment unit 122.

In the embodiments described above, an example is described in which the incident-side pressing portion 226 and the emission-side pressing portion 241 respectively press the end portion on the incident side and the end portion on the emission side of the outer peripheral surface of the layered structure. However, the positions of the layered structure pressed by the pressing portions are not limited to these end portions. For example, the pressing portion may press a central region of the outer peripheral surface of the layered structure.

In the above-described embodiments, an example is described in which the first coils and the second coils generate magnetic fields in directions substantially orthogonal to each other. However, the configuration is not limited thereto, and the first coils and the second coils may generate magnetic fields in directions shifted from directions orthogonal to each other within a range in which the charged particle beam can be scanned.

In the embodiments described above, an example is described in which the shapes of the inner tube 30, the layers constituting the layered structure 42, and the outer tube 32 are circular in a cross section orthogonal to the X-axis. However, these shapes are not limited to circular shapes, and may be various shapes such as elliptical or rectangular shapes.

In the embodiments described above, an example is described in which the entire length of the outer tube 32 in the X-axis direction is about the same as the entire length of the structure 40 in the X-axis direction. However, the lengths are not limited thereto, and the entire length of the outer tube in the X-axis direction may be different from the entire length of the structure in the X-axis direction. For example, the entire length of the outer tube in the X-axis direction may be longer than the entire length of the structure in the X-axis direction, and the outer tube may protrude outward from the end surface of the structure. In this case, the structure 40 may also be pressed by pressing the protruding portion of the outer tube from the exterior toward the interior by the pressing portion.

Further, in the embodiments described above, an example is described in which each of the outflow member 22 and the inflow member 24 is constituted by a combination of a plurality of members. However, the configuration is not limited thereto, and each of the outflow member 22 and the inflow member 24 may be constituted by a single member.

### Industrial Applicability

The present invention can be applied to a charged particle beam irradiation device.

### Reference Signs List

1 Charged particle beam irradiation device, 14 Deflecting electromagnet, 16 Irradiation nozzle, 20 Charged particle beam deflection device, 22 Outflow member, 24 Inflow member, 30 Inner tube, 32 Outer tube, 40 Structure, 42 Layered structure, 43 End surface, 44 End surface, 50 First coil, 52 Second coil, 222 Incident-side protruding portion, 224 Incident-side covering portion, 226 Incident-side pressing portion, 240 Emission-side end portion, 241 Emission-side pressing portion, 243 Emission-side protruding portion, 244 Hole, 300 Internal space, 310, 320 Inner tube protruding portion

## Claims

1. A charged particle beam deflection device, comprising:
a layered structure having a hollow shape where a charged particle beam passes through an interior of the layered structure and including a plurality of layers layered in a direction from the interior toward an exterior of the layered structure;
a first coil configured to deflect the charged particle beam in a first direction different from a travel direction of the charged particle beam;
a second coil configured to deflect the charged particle beam in a second direction different from the first direction; and
a pressing portion located on an outer peripheral surface of the layered structure and configured to press the outer peripheral surface from the exterior toward the interior, wherein
the plurality of layers include
a first coil support layer supporting the first coil and
a second coil support layer supporting the second coil, and
the layered structure includes a gap, through which a cooling solvent flows, between two adjacent layers among the plurality of layers.

2. The charged particle beam deflection device according to claim 1, wherein
the pressing portion includes an annular member located on the outer peripheral surface of the layered structure, extending around the layered structure in a peripheral direction.

3. The charged particle beam deflection device according to claim 1 or 2, wherein
the layered structure includes
an end portion on an incident side of the charged particle beam and
an end portion on an emission side of the charged particle beam, and
the pressing portion presses an outer peripheral surface of one of the end portions of the layered structure from the exterior toward the interior.

4. The charged particle beam deflection device according to claim 3, further comprising:
a covering member covering an end surface of the one of the end portions.

5. The charged particle beam deflection device according to claim 4, wherein
the pressing portion includes a protruding portion fixed to the covering member, the protruding portion protruding outward from the end surface, and
a space is formed surrounded by the end surface and the covering member.

6. The charged particle beam deflection device according to claim 5, further comprising:
a closing portion configured to close an opening on the interior side between the covering member and the end surface included in the space, and
the space communicates with the gap and forms part of a flow path of the cooling solvent.

7. The charged particle beam deflection device according to claim 6, wherein
the pressing portion includes a hole open in a direction parallel to the travel direction of the charged particle beam and communicating with the space, and
the hole forms part of the flow path of the cooling solvent.
